# DEMANDE DE BREVET EUROPEEN

(11) **EP 1 331 000 A1**
(43) Date de publication de la demande: **30.07.2003**
(21) Numéro de dépôt: 03290032.6
(22) Date de dépôt: 07.01.2003
(51) Int. Cl.: A61K 7/42

(54) **Composition de protection solaire de la peau ou des cheveux contenant un polymère semi-cristallin**

(30) Priorité: 24.01.2002 FR 0200882
(71) Demandeur: L'OREAL, 75008 Paris (FR)
(72) Inventeur: Lorant, Raluca, 94320 Thiais (FR); Lennon, Paula, 69003 Lyon (FR)
(74) Mandataire: Rasson, Catherine

(57) **Abrégé**

La présente demande se rapporte à l'utilisation d'un polymère semi-cristallin, dans une composition à application topique destinée à la protection de la peau et/ou des cheveux contre les rayonnements UV, en particulier le rayonnement solaire, contenant au moins un filtre UV organique, comme agent permettant d'augmenter le facteur de protection solaire (SPF). En outre, le polymère semi-cristallin de l'invention permet d'apporter une meilleure dispersion des filtres et confère à la composition une bonne résistance à l'eau (effet waterproof) et de meilleures qualités d'étalement.

Elle se rapporte aussi à une composition à application topique pour la photoprotection des matières kératiniques, en particulier pour la protection de la peau et/ou des cheveux contre le rayonnement solaire, comprenant, dans un support physiologiquement acceptable, au moins un filtre UV organique et une phase grasse contenant au moins un polymère semi-cristallin, le filtre UV organique n'étant pas un dérivé de dibenzoylméthane.

## Description

La présente demande se rapporte à l'utilisation d'un polymère semi-cristallin, dans et/ou pour la fabrication d'une composition à application topique destinée à la protection de la peau et/ou des cheveux contre les rayonnements UV, en particulier le rayonnement solaire, contenant au moins un filtre UV organique, comme agent permettant d'augmenter le facteur de protection solaire (SPF). Elle se rapporte aussi à une composition à application topique contenant un filtre UV et un polymère semi-cristallin, en vue d'améliorer l'indice de protection dans le rayonnement ultraviolet, apporté par les filtres chimiques.

On sait que les rayons lumineux de longueurs d'onde comprises entre 280 nm et 400 nm permettent le brunissement de l'épiderme humain et que, par ailleurs, les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Pour filtrer les rayons UV-A et UV-B, il existe sur le marché différents types de filtres solaires : les pigments et les filtres chimiques (ou filtres UV organiques). Ces filtres solaires doivent pouvoir absorber ou bloquer les rayons nocifs du soleil tout en restant inoffensifs pour l'utilisateur.

Pour des raisons de stabilité de ces compositions et/ou de toxicité sur la peau ou les muqueuses, il n'est pas toujours possible d'augmenter les quantités des filtres chimiques pour augmenter l'indice de protection des compositions solaires. Aussi la demanderesse a cherché un autre moyen pour augmenter l'indice de protection de ces compositions.

Par ailleurs, l'efficacité des filtres chimiques ou physiques est souvent limitée par les problèmes de dispersion de ces filtres dans les compositions cosmétiques, l'hétérogénéité du film formé à la surface de la peau et la faible résistance à l'eau.

En outre, beaucoup de produits, bien qu'agréables lors de l'application sur la peau, présentent l'inconvénient de ne pas bien résister à l'eau, d'où une perte d'efficacité de la protection dans le temps, en particulier en milieu humide, en présence d'eau, de sueur, ou de sable par exemple. Pour répondre à ce problème, les produits solaires sont souvent formulés avec un taux important d'huiles ou de cires pour apporter un effet waterproof. L'inconvénient de ce type de produit est d'apporter un toucher gras, désagréable à l'utilisation.

De façon surprenante, la demanderesse a trouvé qu'en associant les filtres avec un polymère semi-cristallin, on obtenait non seulement une bonne résistance à l'eau mais aussi une amélioration du pouvoir filtrant des filtres et, de ce fait, une nette amélioration de l'indice de protection (SPF ou IP) de la composition les contenant, et ce quelle que soit la forme galénique : aussi bien les gels anhydres, que les émulsions H/E et E/H.

L'utilisation de ce polymère permet de résoudre le problème de mauvaise résistance à l'eau dans le cas des émulsions. En outre, dans toutes les formes galéniques, l'ajout d'un faible taux de ce polymère permet d'obtenir des indices de protection plus élevés sans augmenter le taux de filtres chimiques. Par ailleurs, l'addition de ce polymère permet aussi une meilleure dispersion des filtres.

Les compositions selon l'invention contenant ces polymères permettent d'obtenir des textures nettement améliorées et un moindre risque d'inconfort par rapport aux formulations classiques riches en filtres (le terme « riche » voulant dire au moins 7 % en poids de filtres).

L'invention a pour objet l'utilisation cosmétique d'un polymère semi-cristallin solide à température ambiante et ayant une température de fusion inférieure à 70°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 2 000, dans une composition cosmétique contenant au moins une phase grasse liquide et au moins un filtre UV organique, pour améliorer le pouvoir photoprotecteur de la dite composition.

L'invention a aussi pour objet l'utilisation d'un polymère semi-cristallin solide à température ambiante et ayant une température de fusion inférieure à 70°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 2 000, pour la fabrication d'une composition à application topique contenant au moins une phase grasse liquide et au moins un filtre UV organique, et destinée à la protection de la peau, des lèvres et/ou des cheveux contre les effets néfastes des rayonnements UV, pour améliorer la protection apportée par ladite composition.

On entend dans la présente demande par « température ambiante » une température de 25°C.

Le pouvoir photoprotecteur est représenté par l'indice de protection (IP) qui représente le pouvoir filtrant dans le domaine des UVA. La détermination de l'indice de protection UVA est basée sur la méthode d'évaluation de la pigmentation immédiate et persistante induite par les UVA (Persistant Pigment Darkening : PPD), décrite par Chardon et col. (Method for the UVA protection assessment of sunscreens based on residual immediate pigment darkening. 20th Annual Meeting of the American Society for Photobiology, Marco island, Florida (USA), June 20-24, 1992).

Non seulement, le polymère semi-cristallin améliore l'indice de protection mais il a aussi l'avantage de conférer à la composition de meilleures qualités sensorielles d'étalement et permet l'obtention d'un effet mat et waterproof.

L'invention a encore pour objet une composition à application topique, en particulier pour la photoprotection des matières kératiniques telles que la peau et/ou les cheveux, caractérisée par le fait qu'elle comprend, dans un milieu physiologiquement acceptable, (1) au moins une phase grasse liquide, (2) au moins un filtre UV organique et (3) au moins un polymère semi-cristallin solide à température ambiante et ayant une température de fusion inférieure à 70°C comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 2 000, et le filtre UV n'étant pas un dérivé de dibenzoylméthane.

Les compositions de l'invention étant destinées à une application topique contiennent un milieu physiologiquement acceptable, c'est-à-dire un milieu compatible avec toutes les matières kératiniques telles que la peau, les ongles, les muqueuses et les cheveux ou toute autre zone cutanée du corps.

La composition peut constituer notamment une composition cosmétique. De manière générale, une composition cosmétique est destinée à être mise en contact avec les parties superficielles du corps humain. Une composition cosmétique solaire permet de lutter contre les effets des rayons UV sur les couches superficielles de la peau et notamment contre les effets sur le vieillissement de la peau (rides et ridules).

Le polymère semi-cristallin utilisé dans la composition de l'invention permet d'obtenir des indices de protection plus élevés sans augmenter le taux de filtres chimiques, donc d'améliorer l'indice de protection pour une quantité de filtre déterminée. Il est généralement introduit dans la phase grasse liquide (appelée aussi ci-après phase huileuse).

Par "polymère semi-cristallin", on entend au sens de l'invention, des polymères comportant une partie cristallisable, chaîne pendante ou séquence dans le squelette, et une partie amorphe dans le squelette et présentant une température de changement de phase réversible du premier ordre, en particulier de fusion (transition solide-liquide). Par "polymères", on entend au sens de l'invention, des composés comportant au moins 2 motifs répétitifs, de préférence au moins 3 motifs répétitifs et plus spécialement au moins 10 motifs répétitifs. Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

De façon avantageuse, le ou les polymères semi-cristallins de la composition de l'invention ont une masse moléculaire moyenne en nombre Mn supérieure ou égale à 2000, allant par exemple de 2 000 à 800 000, de préférence de 3 000 à 500 000, par exemple de 4 000 à 150 000, et mieux de 4 000 à 99 000.

Dans la composition selon l'invention les polymères semi-cristallins sont avantageusement solubles dans la phase grasse à au moins 1 % en poids, à une température supérieure à leur température de fusion. En dehors des chaînes ou séquences cristallisables, les séquences des polymères sont amorphes. Par "chaîne ou séquence cristallisable", on entend au sens de l'invention une chaîne ou séquence qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère.

De préférence, le squelette polymérique des polymères semi-cristallins est soluble dans la phase grasse liquide.

De façon préférée, les polymères semi-cristallin utilisés dans la composition de l'invention présentent une température de fusion (ou point de fusion) pF inférieure à 70°C, de préférence inférieure à 50°C, cette température étant au moins égale à la température du support kératinique devant recevoir la composition selon l'invention. Le polymère semi-cristallin a une température de fusion pF telle que 25°C ≤ pF< 70°C et de préférence 30°C ≤ pF < 50°C. La température de fusion peut être mesurée notamment par toute méthode connue et en particulier avec un calorimètre à balayage différentiel (D.S.C).

De préférence, les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères séquencés ou multiséquencés. Ils peuvent être obtenus par polymérisation de monomères à doubles liaisons réactives (liaisons éthyléniques) ou par polycondensation. Lorsque les polymères de l'invention sont des polymères à chaînes latérales cristallisables, ceux-ci sont avantageusement sous forme aléatoire ou statistique.

De préférence, les polymères semi-cristallins de l'invention sont d'origine synthétique. En outre, ils ne comportent pas de squelette polysaccharidique.

Les polymères semi-cristallins utilisables dans l'invention sont en particulier :
1. les copolymères séquencés de polyoléfines à cristallisation contrôlée, dont les monomères sont décrits dans le document EP-A-951897 ;
2. les polycondensats et notamment les polycondensats polyesters, aliphatiques ou aromatiques, et les copolyesters aliphatiques/aromatiques ;
3. les homo- ou co-polymères portant au moins une chaîne latérale cristallisable et les homo- ou co-polymères portant dans le squelette au moins une séquence cristallisable, comme ceux décrits dans le document US-A-5,156,911 ;
4. les homo- ou co-polymères portant au moins une chaîne latérale cristallisable à groupement(s) fluoré(s), tels que décrits dans le document WO-A-01/19333 ;
5. et leurs mélanges.

Dans les deux derniers cas (3 et 4), la ou les chaînes latérales ou séquences cristallisables sont hydrophobes.

Les polymères cristallins à chaînes latérales cristallisables ou portant dans le squelette au moins une séquence cristallisable sont décrits ci-dessous.

### A) Polymères semi-cristallins à chaînes latérales cristallisables

On peut citer en particulier ceux définis dans les documents US-A-5,156,911 et WO-A-01/19333. Ce sont des homopolymères ou copolymères comportant de 50 à 100 % en poids de motifs résultant de la polymérisation d'un ou de plusieurs monomères porteurs de chaîne(s) latérale(s) hydrophobe(s) cristallisable(s).
Ces homo- ou co-polymères sont de toute nature du moment qu'ils présentent les conditions indiquées ci-après avec, en particulier, la caractéristique d'être solubles ou dispersables dans la phase grasse liquide par chauffage au-dessus de leur température de fusion pF. Ils peuvent résulter :
- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique ;
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

a) D'une façon générale les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule X : 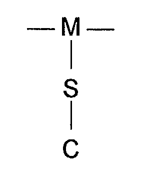
où M représente un atome du squelette polymérique, S représente un espaceur et C représente un groupe cristallisable.

Les chaînes « -S-C» cristallisables peuvent être aliphatiques ou aromatiques, éventuellement fluorées ou perfluorées. « S » représente notamment un groupe (CH₂)ₙ ou (CH₂CH₂O)ₙ ou (CH₂O), linéaire ou ramifié ou cyclique, n étant un nombre entier allant de 0 à 22. De préférence « S » est un groupe linéaire. De préférence, « S » et « C » sont différents.

Lorsque les chaînes cristallisables sont des chaînes aliphatiques (alkyle), elles comportent au moins 11 atomes de carbone et au plus 40 atomes de carbone et mieux au plus 24 atomes de carbone. Il s'agit notamment de chaînes alkyle possédant au moins 12 atomes de carbone, et de préférence, il s'agit de chaînes alkyle comportant de 14 à 24 atomes de carbone (C₁₄-C₂₄). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés.

Comme exemples de polymères ou copolymères semi-cristallins à chaîne(s) cristallisable(s), on peut citer ceux résultant de la polymérisation d'au moins un monomère à chaîne cristallisable choisi parmi les (méth)acrylates d'alkyle saturés en C₁₄-C₂₄ (C₁₄-C₂₄ signifie que le groupe alkyle comporte de 14 à 24 atomes de carbone) ; les (méth)acrylates de perfluoroalkyle en C₁₁-C₁₅ (groupe alkyle avec 11 à 15 atomes de carbone) ; les N-alkyl (méth)acrylamides en C₁₄ à C₂₄ avec ou sans atome de fluor (groupe alkyle avec 14 à 24 atomes de carbone) ; les esters vinyliques à chaînes alkyle ou perfluoroalkyle en C₁₄ à C₂₄ (groupe alkyle avec 14 à 24 atomes de carbone), avec une chaîne perfluoroalkyle comportant au moins 6 atomes de fluor ; les éthers vinyliques à chaînes alkyle ou perfluoroalkyle en C₁₄ à C₂₄ (groupe alkyle avec 14 à 24 atomes de carbone), avec une chaîne perfluoroalkyle comportant au moins 6 atomes de fluor ; les alpha-oléfines en C₁₄ à C₂₄ (groupe alkyle avec 14 à 24 atomes de carbone), comme par exemple l'octadécène ; les para-alkyl styrènes en C₁₄ à C₂₄ (groupe alkyle avec 14 à 24 atomes de carbone), leurs mélanges.

Par "alkyle", on entend au sens de l'invention un groupement saturé notamment comportant de 8 à 24 atomes de carbone (C₈ à C₂₄), sauf mention exprès.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères utilisés dans la composition de l'invention sont des copolymères, ils contiennent, en plus, de 0 à 50 % de groupes Y ou Z résultant de la copolymérisation :
α ) avec Y qui est un monomère polaire ou non polaire ou un mélange des deux :
   - Lorsque Y est un monomère polaire, c'est soit un monomère porteur de groupes polyoxyalkylénés (notamment oxyéthylénés et/ou oxypropylénés), un (méth)acrylate d'hydroxyalkyle comme l'acrylate d'hydroxyéthyle, le (méth)acrylamide, un N-alkyl(méth)acrylamide, un NN-dialkyl(méth)acrylamide comme par exemple le NN-diisopropylacrylamide ou la N-vinyl-pyrrolidone (NVP), le N-vinyl-caprolactame, soit un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   - Lorsque Y est un monomère non polaire, il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle comportant de 1 à 10 atomes de carbone (C₁ à C₁₀), comme l'α-méthylstyrène, un macromonomère du type polyorganosiloxane à insaturation vinylique.
β) avec Z qui est un monomère polaire ou un mélange de monomères polaires, Z ayant la même définition que le "Y polaire" défini ci-dessus.

De préférence, les polymères semi-cristallins à chaîne latérale cristallisable sont choisis parmi les homopolymères d'alkyl(méth)acrylate ou d'alkyl(méth)acrylamide avec un groupe alkyle tel que défini ci-dessus, et notamment en C₁₄-C₂₄ ; les copolymères de ces monomères avec un monomère hydrophile de préférence de nature différente de l'acide (méth)acrylique ; et leurs mélanges. Il peut s'agir par exemple comme copolymères, des copolymères d'alkyl(méth)acrylate ou d'alkyl (méth)acrylamide avec un groupe alkyle en C₁₄ à C₂₄, avec la N-vinylpyrrolidone, l'hydroxyéthyl (méth)acrylate ; ou leurs mélanges.

### B) Les polymères portant dans le squelette au moins une séquence cristallisable

II s'agit encore de polymères solubles ou dispersables dans la phase grasse liquide par chauffage au-dessus de leur point de fusion pF. Ces polymères sont notamment des copolymères séquencés constitués d'au moins 2 séquences de nature chimique différente dont l'une est cristallisable.

On peut utiliser :
1) Les polymères définis dans le document US-A-5,156,911 ;
2) Les copolymères séquencés d'oléfine ou de cyclooléfine à chaîne cristallisable comme ceux issus de la polymérisation séquencée de :
   - cyclobutène, cyclohexène, cyclooctène, norbornène (c'est-à-dire bicyclo(2,2,1)heptène-2), 5-méthylnorbornène, 5-éthylnorbornène, 5,6-diméthyl-norbornène, 5,5,6-triméthyl norbornène, 5-éthylidène-norbornène, 5-phényl-norbornène, 5-benzylnorbornène, 5-vinylnorbornène, 1,4,5,8-diméthano-1,2,3,4,4a,5,8a-octahydronaphtalène, dicyclopentadiène, ou leurs mélanges ;
   - avec l'éthylène, le propylène, le 1-butène, le 3-méthyl-1-butène, le 1-hexène, le 4-méthyl-1-pentène, le 1-octène, le 1-décène, le 1-éicosène ou leurs mélanges.

   Ces copolymères séquencés peuvent être en particulier les copolymères (éthylène/norbornène) blocs et les terpolymères blocs (éthylène/propylène/éthylidène-norbornène).
   On peut aussi utiliser ceux résultants de la copolymérisation séquencée d'au moins 2 α-oléfines en C₂-C₁₆ et mieux en C₂-C₁₂, tels que ceux cités précédemment et en particulier les bipolymères séquencés d'éthylène et d'1-octène.
3) Les copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
   . Séquence cristallisable par nature : a) polyester comme les poly(alkylène téréphtalate), b) polyoléfine comme les polyéthylènes ou polypropylènes.
   . Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :
α) les copolymères séquencés poly(ε-caprolactone)-b-poly(butadiène), utilisés de préférence hydrogénés, tels que ceux décrits dans l'article "Melting behavior of poly(ε-caprolactone)-block-polybutadiène copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993), et "Polymer agregates with crystalline cores : the system poly(ethylene)-poly(ethylene-propylene)" P. Richter et al., Macromolécules, 30, 1053-1068 (1997).
δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être non réticulés ou réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins de la composition selon l'invention sont non réticulés.

A titre d'exemple particulier de polymère semi-cristallin utilisable dans la composition selon l'invention, on peut citer les produits Intelimer® de la société Landec décrits dans la brochure "Intelimer® polymers". Ces polymères sont sous forme solide à température ambiante (25°C). Ils sont porteurs de chaînes latérales cristallisables et présentent le monomère de formule X précédente. On peut citer notamment le « Landec IP22 », ayant une température de fusion pF de 56°C, qui est un produit visqueux à température ambiante, imperméable, non-collant.

On peut aussi utiliser les polymères semi-cristallins décrits dans les exemples 3, 4, 5, 7, 9 du document US-A-5,156,911, résultant de la copolymérisation d'acide acrylique et d'alkyl(méth)acrylate en C₅ à C₁₆ ayant un pF allant de 20°C à 35°C, et plus particulièrement ceux résultant de la copolymérisation :
. d'acide acrylique, d'hexadécylacrylate et d'isodécylacrylate dans un rapport 1/16/3,
. d'acide acrylique et de pentadécylacrylate dans un rapport 1/19,
. d'acide acrylique, d'hexadécylacrylate, d'éthylacrylate dans un rapport 2,5/76,5/20,
. d'acide acrylique, d'hexadécylacrylate et de méthylacrylate dans un rapport 5/85/10,
. d'acide acrylique, d'octadécylméthacrylate dans un rapport 2,5/97,5.

On peut aussi utiliser le polymère « Structure O » commercialisé par la société National Starch, tel que celui décrit dans le document US-A-5,736,125, de pF 44°C, ainsi que les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP, tels que décrits dans le document US-A-5,519,063 ou EP-A- 0550745, et plus spécialement ceux décrits dans les exemples 1 et 2, ci-après, de préparation de polymère, de température de fusion respectivement de 40°C et 38°C.

On peut aussi utiliser les polymères semi-cristallins obtenus par copolymérisation de l'acrylate de béhényle et de l'acide acrylique ou de NVP, tels que décrits dans les documents US-A-5,519,063 et EP-A- 0 550745, et plus spécialement ceux décrits dans les exemples 3 et 4, ci-après, de préparation de polymère, de température de fusion respectivement de 60°C et 58°C.

Selon un mode particulier de réalisation de l'invention, les polymères semi-cristallins utilisés ne comportent pas de groupement carboxylique.

La quantité de polymère semi-cristallin dans la composition de l'invention peut varier dans une large mesure suivant le but recherché et notamment une gélification plus ou moins grande de la phase huileuse. La quantité de polymères semi-cristallins peut aller par exemple de 0,1 à 50 % en poids de matière active, de préférence de 0,5 à 20 % en poids de matière active et mieux de 1 à 10 % en poids de matière active par rapport au poids total de la composition.

### Filtres UV organiques (ou filtres solaires)

La composition de l'invention contient au moins un filtre UV organique choisi parmi les filtres organiques hydrophiles, les filtres organiques lipophiles et leurs mélanges. Selon un mode particulier de réalisation de l'invention, on peut y associer un ou plusieurs filtres physiques.

Comme exemples de filtres organiques, actifs dans l'UV-A et/ou l'UV-B, pouvant être utilisés dans la composition de l'invention, on peut citer par exemple, désignés ci-dessus sous leur nom CTFA :

### Dérivés de l'acide para-aminobenzoique (PABA) :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques :

- Homosalate vendu sous le nom « EUSOLEX HMS » par RONA/EM INDUSTRIES,
- Ethylhexyl Salicylate (ou salicylate d'éthyl hexyle) vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

### Dérivés du dibenzoylméthane:

- Butyl Methoxydibenzoylmethane vendu notamment sous le nom commercial « PARSOL 1789 » par HOFFMANN LA ROCHE,
- Isopropyl Dibenzoylmethane,

### Dérivés cinnamiques :

- Ethylhexyl Methoxycinnamate (ou Octyl Methoxycinnamate) vendu notamment sous le nom commercial « PARSOL MCX » par HOFFMANN LA ROCHE,
- Isopropyl Methoxycinnamate,
- Isoamyl Methoxycinnamate vendu sous le nom commercial « NEO HELIOPAN E 1000 » par HAARMANN et REIMER,
- Cinoxate,
- DEA Methoxycinnamate,
- Diisopropyl Methylcinnamate,
- Glyceryl Ethylhexanoate Dimethoxycinnamate

### Dérivés de β,β-diphénylacrylate:

- Octocrylene (α-cyano-β,β-diphénylacrylate de 2-éthylhexyle) vendu notamment sous le nom commercial « UVINUL N539 » par BASF,
- Etocrylene, vendu notamment sous le nom commercial « UVINUL N35 » par BASF,

### Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

### Dérivés du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- Terephthalylidene Dicamphor Sulfonic Acid fabriqué sous le nom « MEXORYL SX » par CHIMEX,
- Polyacrylamidomethyl Benzylidene Camphor fabriqué sous le nom « MEXORYL SW » par CHIMEX,

### Dérivés du phenyl benzimidazole :

- Phenylbenzimidazole Sulfonic Acid vendu notamment sous le nom commercial « EUSOLEX 232 » par MERCK,
- Benzimidazilate vendu sous le nom commercial « NEO HELIOPAN AP » par HAARMANN et REIMER,

### Dérivés de la triazine :

- Anisotriazine vendu sous le nom commercial «TINOSORB S » par CIBA GEIGY,
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,

### Dérivés du phenyl benzotriazole :

- Drometrizole Trisiloxane vendu sous le nom « SILATRIZOLE » par RHODIA CHIMIE,

### Dérivés anthraniliques :

- Menthyl anthranilate vendu sous le nom commercial « NEO HELIOPAN MA » par HAARMANN et REIMER,

### Dérivés d'imidazolines :

- Ethylhexyl Dimethoxybenzylidene Dioxoimidazoline Propionate,

### Dérivés du benzalmalonate :

- Polyorganosiloxane à fonctions benzalmalonate vendu sous la dénomination commerciale « PARSOL SLX » par HOFFMANN LA ROCHE
- et leurs mélanges.

Les filtres UV organiques plus particulièrement préférés sont choisis parmi les composés suivants :
- Ethylhexyl salicylate,
- Butyl Methoxydibenzoylmethane,
- Ethylhexyl Methoxycinnamate,
- Octocrylène,
- Phenylbenzimidazole Sulfonic Acid (acide phenylbenzimidazole sulfonique),
- Terephthalylidene Dicamphor Sulfonic Acid (acide téréphthalylidène dicamphosulfonique),.
- Benzophenone-3,
- Benzophenone-4,
- Benzophenone-5,
- 4-Methylbenzylidene camphor (4-méthylbenzylidène camphre),
- Benzimidazilate,
- Anisotriazine,
- Ethylhexyl triazone,
- Diethylhexyl Butamido Triazone,
- Methylene bis-Benzotriazolyl Tetramethylbutylphenol (méthylène bis-benzotriazolyl tétraméthylbutylphenol),
- Drometrizole Trisiloxane,
- et leurs mélanges.

Le ou les filtres organiques peuvent être présents en une quantité allant de 0,1 à 25 % en poids, de préférence de 1 à 20 % en poids, et mieux 5 à 15 % en poids par rapport au poids total de la composition.

Comme filtres physiques pouvant être ajoutés dans la composition de l'invention, on peut citer par exemple les pigments et nano-pigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

Selon l'invention, les nanopigments peuvent être traités ou non traités en surface et peuvent être choisis notamment parmi les nanopigments d'oxydes de titane (amorphe ou cristallisé sous forme rutile et/ou anatase), de fer, de zirconium, de zinc, de cérium et leurs mélanges, enrobés ou non.

Les nanopigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les nanopigments traités peuvent être plus particulièrement des oxydes de titane traités par :
- la silice et l'alumine tels que les produits « Microtitanium Dioxide MT 500 SA » et « Microtitanium Dioxide MT 100 SA » de la société TAYCA, et les produits « Tioveil Fin », « Tioveil OP », « Tioveil MOTG » et « Tioveil IPM » de la société TIOXIDE,
- l'alumine et le stéarate d'aluminium tels que le produit « Microtitanium Dioxide MT 100 T » de la société TAYCA,
- l'alumine et le laurate d'aluminium tels que le produit « Microtitanium Dioxide MT 100 S » de la société TAYCA,
- des oxydes de fer et le stéarate de fer tels que le produit « Microtitanium Dioxide MT 100 F » de la société TAYCA,
- la silice, l'alumine et la silicone tels que les produits « Microtitanium Dioxide MT 100 SAS », « Microtitanium Dioxide MT 600 SAS » et « Microtitanium Dioxide MT 500 SAS » de la société TAYCA,
- l'hexamétaphosphate de sodium tels que le produit « Microtitanium Dioxide MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212 » de la société KEMIRA,
- l'alumine et la silicone tels que le produit « UVT-M262 » de la société KEMIRA.

Les oxydes de titane non traités peuvent par exemple être ceux vendus par la société TAYCA sous les dénominations commerciales « Microtitanium Dioxide MT 500 B » ou « Microtitanium Dioxide MT 600 B ».

Les oxydes de zinc non traités peuvent par exemple être ceux vendus par la société SUMITOMO sous la dénomination « Ultra Fine Zinc Oxide Powder », par la société PRESPERSE sous la dénomination « Finex 25 », par la société IKEDA sous la dénomination « MZO-25 » ou par la société SUNSMART sous la dénomination « Z-COTE ». Les oxydes de zinc traités peuvent par exemple être ceux vendus par la société SUNSMART sous la dénomination « Z-COTE HP 1 ».

Les nanopigments peuvent être introduits dans les compositions selon l'invention tels quels ou sous forme de pâte pigmentaire, c'est-à-dire en mélange avec un dispersant, comme décrit par exemple dans le document GB-A-2206339.

Le ou les filtres physiques peuvent être présents dans les compositions selon l'invention dans une proportion allant de 0,1 à 20 % en poids, de préférence de 0,5 à 12 % en poids, et mieux de 1 à 5 % en poids par rapport au poids total de la composition.

La phase grasse contenant le polymère semi-cristallin contient habituellement au moins une huile, à savoir une substance organique liquide à la température ambiante (20 à 25°C) et à la pression atmosphérique (760 mm Hg). Comme huiles cosmétiques utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle ; les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de FLUTEC PC1® et FLUTEC PC3® par la Société BNFL Fluorochemicals ; le perfluoro-1,2-dlméthylcyclobutane ; les perfluoroalcanes tels que le dodécafluoropentane et le tétradécafluorohexane, vendus sous les dénominations de PF 5050® et PF 5060® par la Société 3M, ou encore le bromoperfluorooctane vendu sous la dénomination FORALKYL® par la Société Atochem ; le nonafluorométhoxybutane vendu sous la dénomination MSX 4518® par la Société 3M et le nonafluoroéthoxyisobutane ; les dérivés de perfluoromorpholine, tels que la 4-trifluorométhyl perfluoromorpholine vendue sous la dénomination PF 5052® par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les huiles végétales hydrogénées comme l'huile de jojoba hydrogénée, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries, ainsi que les élastomères de silicone comportant une ou plusieurs chaînes oxyalkylénés et notamment oxyéthylénés, tels que le produit commercialisé sous la dénomination « KSG 21 » par la société Shin-Etsu ; et leurs mélanges.

Ces corps gras peuvent être choisis de manière variée par l'homme du métier afin de préparer une composition ayant les propriétés, par exemple de consistance ou de texture, souhaitées.

Les compositions utilisées selon l'invention peuvent se présenter sous toutes les formes galéniques classiquement utilisées pour une application topique et notamment sous forme de solutions huileuses, d'émulsions huile-dans-eau (H/E) ou eau-dans-huile (E/H) ou multiple, de gels huileux, de produits anhydres liquides, pâteux ou solides, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent aussi être colorées quand elles sont utilisées comme produits de maquillage, généralement par addition de pigments colorés ou de colorants. Elles peuvent être éventuellement appliquées sur la peau sous forme d'aérosol. Elles peuvent aussi se présenter sous forme solide, et par exemple sous forme de stick.

Quand la composition selon l'invention est une émulsion eau-dans-huile (E/H) ou huile-dans-eau (H/E),.la proportion de la phase grasse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90^{R} par la société Goldschmidt et le mélange de cétyl diméthicone copolyol, d'isostearate de polyglycérole (4 moles) et de laurate d'hexyle vendu sous la dénomination ABIL WE O9 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Les compositions de l'invention peuvent contenir également tous les adjuvants classiquement utilisés dans le domaine cosmétique ou dermatologique, dans les concentrations habituelles. Ces adjuvants sont en particulier choisis parmi les gélifiants hydrophiles ou lipophiles, les conservateurs, les opacifiants, les émulsionnants, les coémulsionnants, les neutralisants, les parfums et leurs solubilisants ou peptisants, les matières colorantes, les charges, ainsi que les actifs lipophiles ou hydrophiles. Ces adjuvants sont présents dans des quantités allant de préférence de 0,01 à 30 % du poids de la composition.

Comme gélifiants hydrophiles, on peut citer par exemple les polymères carboxyvinyliques tels que les carbopols (carbomers) et les Pemulen (Copolymère acrylate/C₁₀-C₃₀-alkylacrylate) ; les polyacrylamides comme par exemple les copolymères réticulés vendus sous les noms Sepigel 305 (nom C.T.F.A. :
polyacrylamide/C13-14 isoparaffin/Laureth 7) ou Simulgel 600 (nom C.T.F.A. :
   acrylamide / sodium acryloyldimethyltaurate copolymer / isohexadecane /polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et
   notamment les gommes telles que la gomme de xanthane ; et leurs mélanges.

Comme gélifiants lipophiles, on peut citer les argiles modifiées telles que le l'hectorite et ses dérivés, comme les produits commercialisés sous les noms de Bentone.

Bien entendu, ces adjuvants doivent être de nature et en concentration telles qu'ils ne diminuent pas l'indice de protection de la composition et ne déstabilisent pas cette dernière.

Les compositions selon l'invention permettent une bonne protection, et notamment une bonne photoprotection (protection solaire) de la peau et/ou des cheveux, et par voie de conséquence, ont une action sur le vieillissement photo-induit de la peau ainsi que sur les rides et/ou ridules de la peau induites par le rayonnement UV et notamment le rayonnement solaire, et sur les effets néfastes pour la santé de la peau des rayonnements UV et notamment du rayonnement solaire.

Aussi, l'invention se rapporte également à l'utilisation d'une composition telle que définie ci-dessus, pour la fabrication d'une composition destinée à la protection de la peau et/ou des cheveux contre les effets néfastes des rayonnements U.V., en particulier le rayonnement solaire.

Les exemples qui suivent servent à illustrer l'invention sans toutefois présenter un caractère limitatif. Dans ces exemples, les compositions sont données en % pondéral sauf mention contraire.

### I) Exemples de fabrication de polymères semi-cristallins

### Exemple 1 : Polymère acide de point de fusion de 40°C

Dans un réacteur d'1l muni d'une agitation centrale avec ancre, d'un réfrigérant et d'un thermomètre, on introduit 120 g d'huile de Parléam® (huile minérale) que l'on chauffe de la température ambiante à 80°C en 45 min. A 80°C, on introduit en 2h le mélange C₁ suivant :
40 g de cyclohexane + 4 g de Triganox 141 [ 2,5 bis (2-éthyl hexanoyl peroxy) - 2,5 - diméthyl hexane].
30 min après le début de la coulée du mélange C₁, on introduit en 1h30 le mélange C₂ constitué de :
190 g d'acrylate de stéaryle + 10 g d'acide acrylique + 400 g de cyclohexane.
A la fin des deux coulées, on laisse réagir 3h supplémentaires à 80°C puis on distille à pression atmosphérique la totalité du cyclohexane présent dans le milieu réactionnel.
On obtient alors le polymère à 60 % en poids en matière active dans l'huile de Parléam®.
Sa masse moléculaire moyenne en poids M_{w} est de 35 000 exprimée en équivalent polystyrène et sa température de fusion pF est de 40°C ± 1°C, mesurée par D.S.C.

### Exemple 2 : Polymère basique de point de fusion de 38°C

On applique le même mode opératoire que dans l'exemple 1, sauf que l'on utilise de la N-Vinyl Pyrrolidone en lieu et place de l'acide acrylique.

Le polymère obtenu est à 60% en poids en matière active dans l'huile de Parléam®, sa masse moléculaire moyenne en poids M_{w} est de 38 000 et son pF de 38°C.

### Exemple 3 : Polymère Acide de point de fusion de 60°C

On applique le même mode opératoire que dans l'exemple 1, sauf que l'on utilise de l'acrylate de béhényle en lieu et place de l'acrylate de stéaryle. Le polymère obtenu est à 60% en poids en matière active dans l'huile de Parléam®. Sa masse moléculaire moyenne en poids M_{w} est de 42 000 et son pF de 60°C.

### Exemple 4 : Polymère basique de point de fusion de 58°C

On applique le même mode opératoire que dans l'exemple 2, sauf que l'on utilise de l'acrylate de béhényle en lieu et place de l'acrylate de stéaryle. Le polymère obtenu est à 60% en poids en matière active dans l'huile de Parléam®. Son M_{w} est de 45 000 et son pF est de 58°C.

### II) Exemples de composition

### Exemple 5 : Gel frais de SPF 20

- Polymère semi-cristallin de l'exemple 1 5 %
- Ethylhexyl methoxycinnamate (Parsol MCX) 7 %
- Benzophenone-3 2 %
- Isohexadécane (huile) qsp 100 %

Le SPF du gel obtenu, mesuré *in vitro*, est de 20.

### Exemple 6 (comparatif) : Gel de SPF 15

- Ethylhexyl methoxycinnamate (Parsol MCX) 7 %
- Benzophenone-3 2 %
- Isohexadécane (huile) qsp 100 %
le SPF du gel obtenu, mesuré *in vitro*, est de 15, bien que la quantité de filtre soit identique à celle de l'exemple 1.

### Exemple 7 : Emulsion E/H de SPF30 (crème)

### Phase huileuse :

- Polymère semi-cristallin de l'exemple 1 5 %
- Cétyl diméthicone copolyol (Abil EM90 de la société Goldschmidt) 3 %
- Isostéarate de glycérol (Isolan GI34 de la société Goldschmidt) (co-émulsionnant) 1 %
- Huile minérale 5 %
- Cyclométhicone 3 %
- Ethylhexyl méthoxycinnamate (Parsol MCX) 7 %
- Dioxyde de titane enrobé acide stéarique (TIOSPERSE ULTRA de la société Collaborative Laboratories) 10 %

### Phase aqueuse :

Glycérine 5 %
Conservateur qs
Eau qsp 100 %

Mode opératoire : on homogénéise les deux phases à chaud (>80°C), puis on fait l'émulsion sous agitation par dispersion de la phase aqueuse dans la phase huileuse.

On obtient une émulsion stable, sous forme d'une crème agréable à appliquer, ayant un SPF mesuré *in vitro* de 30.

### Exemple 8 : Emulsion gel H/E de SPF 20

### Phase huileuse :

- Polymère semi-cristallin de l'exemple 1 2 %
- Huile minérale 6 %
- Cyclométhicone 3 %
- Ethylhexyl methoxycinnamate (Parsol MCX) 7 %
- Benzophénone - 3 2 %

### Phase aqueuse :

- Glycérine 5 %
- Ammonium polyacryldimethyl tauramide (Hostacerin AMPS de la société Clariant) 1,5 %
- Conservateur qs
- Eau qsp 100 %

Mode opératoire : on fait gonfler le gel d'AMPS dans la phase aqueuse à chaud. Par ailleurs, on homogénéise les composants de la phase huileuse à une température d'environ 80°C, puis on fait l'émulsion sous agitation par dispersion de la phase huileuse dans la phase aqueuse.

On obtient une émulsion qui constitue une crème, fraîche à l'application et agréable à utiliser. Cette crème a un SPF mesuré *in vitro* de 20.

### Exemple 9 : Crème de jour (Emulsion H/E)

### Phase huileuse A :

- Polymère semi-cristallin de l'exemple 1 2 %
- Huile minérale 6 %
- Cyclométhicone 3 %
- Ethylhexyl methoxycinnamate (Parsol MCX) 7 %

### Phase aqueuse B :

- Glycérine 5 %
- Ammonium polyacryldimethyl tauramide (Hostacerin AMPS de la société Clariant) 1,5 %
- Mélange d'arachidyl polyglucoside et d'alcools arachidique et béhénique (15/85) (Montanov 202 de la société Seppic) 3 %
- Stéarate de glycéryle 1 %
- Conservateur qs
- Triéthanolamine 0,4 %
- Acide terephtalylidene-3,3' di-camphosulfonique-10,10' dans l'eau à 33 % (Mexoryl SX de la société Chimex) 2,1 %
- Eau qsp 100 %

### Phase C :

- Acide phenyl-2 benzimidazole sulfonique-5 (Eusolex 232 de la société Rona /EM Industries) 2 %
- Triéthanolamine 1,7 %
- Eau 10 %

Mode opératoire : on fait gonfler le gel d'AMPS dans la phase aqueuse B à 50°C. Par ailleurs, on homogénéise les composants de la phase huileuse à 60°C, puis on fait l'émulsion sous agitation par dispersion de la phase huileuse dans la phase aqueuse. On homogénéise par ailleurs les composants de la phase C jusqu'à dissolution totale du filtre. On ajoute ensuite cette phase C dans l'émulsion (A + B).

On obtient une belle crème blanche, douce et non-grasse au toucher apte à protéger des UVA et UVB. Ce produit peut s'utiliser tous les jours pour protéger des effets néfastes du soleil. *La SPF in-vitro est de 20.*

## Revendications

1. Utilisation cosmétique d'un polymère semi-cristallin solide à température ambiante et ayant une température de fusion inférieure à 70°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 2 000, dans une composition cosmétique destinée à la protection de la peau, des lèvres et/ou des cheveux contre le rayonnement UV et contenant au moins une phase grasse liquide et au moins un filtre UV organique, pour améliorer le pouvoir photoprotecteur de ladite composition.

2. Utilisation d'un polymère semi-cristallin solide à température ambiante et ayant une température de fusion inférieure à 70°C, comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 2 000, pour la fabrication d'une composition à application topique contenant au moins une phase grasse liquide et au moins un filtre UV organique, et destinée à la protection de la peau, des lèvres et/ou des cheveux contre les effets néfastes des rayonnements UV, pour améliorer la protection apportée par ladite composition.

3. Utilisation selon la revendication 1 ou 2, **caractérisée en ce que** le polymère a une masse moléculaire moyenne en nombre allant de 3 000 à 500 000 et mieux de 4 000 à 99 000.

4. Utilisation selon l'une quelconque des revendications précédentes, **caractérisée en ce que** le polymère est soluble dans la phase grasse à au moins 1 % en poids à une température supérieure à sa température de fusion.

5. Utilisation selon l'une quelconque des revendications précédentes, **caractérisée en ce que** le polymère a une température de fusion pF telle que 25°C ≤ pF < 70°C et de préférence 30°C ≤ pF < 50°C.

6. Utilisation selon l'une quelconque des revendications précédentes, **caractérisée en ce que** le polymère est choisi parmi :
- les copolymères séquencés de polyoléfines à cristallisation contrôlée,
- les polycondensats polyesters aliphatiques ou aromatiques et les copolyesters aliphatiques/aromatiques,
- les homo- ou co-polymères portant au moins une chaîne latérale cristallisable, et les homo- ou co-polymères portant dans le squelette au moins une séquence cristallisable,
- les homo- ou co-polymères portant au moins une chaîne latérale cristallisable à groupement(s) fluoré(s),
- et leurs mélanges.

7. Utilisation selon l'une quelconque des revendications précédentes, **caractérisée en ce que** le polymère est choisi parmi les homopolymères et copolymères comportant de 50 à 100% en poids de motifs résultant de la polymérisation d'un ou de plusieurs monomères porteurs de chaîne(s) latérale(s) hydrophobe(s) cristallisable(s).

8. Utilisation selon l'une quelconque des revendications précédentes, **caractérisée en ce que** le polymère est choisi parmi les homopolymères et copolymères résultant de la polymérisation d'au moins un monomère à chaîne(s) cristallisable(s) de formule X : 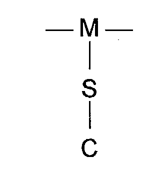 où M représente un atome du squelette polymérique, S représente un espaceur et C représente un groupe cristallisable.

9. Utilisation selon l'une quelconque des revendications précédentes, **caractérisée en ce que** le polymère est choisi parmi les homopolymères et copolymères résultant de la polymérisation d'au moins un monomère à chaîne cristallisable choisi parmi les (méth)acrylates d'alkyle saturés en C₁₄-C₂₄, les (méth)acrylates de perfluoroalkyle en C₁₁-C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, et leurs mélanges.

10. Utilisation selon l'une quelconque des revendications précédentes, **caractérisée en ce que** le polymère est choisi parmi les homopolymères d'alkyl(méth)acrylate ou d'alkyl(méth)acrylamide en C₁₄ à C₂₄ ; des copolymères de ces monomères avec un monomère hydrophile ; et leurs mélanges.

11. Utilisation selon l'une quelconque des revendications précédentes, **caractérisée en ce que** le polymère est choisi parmi les copolymères d'alkyl(méth)acrylate ou d'alkyl (méth)acrylamide avec un groupe alkyle en C₁₄ à C₂₄, avec la N-vinylpyrrolidone, l'hydroxyéthyl (méth)acrylate ; ou leurs mélanges.

12. Utilisation selon l'une quelconque des revendications précédentes, **caractérisée en ce que** le polymère représente de 0,1 à 50 % en poids de matière active, et de préférence de 0,5 à 20 % en poids de matière active par rapport au poids total de la composition.

13. Utilisation selon l'une quelconque des revendications précédentes, **caractérisée en ce que** le filtre UV organique est choisi parmi les filtres organiques hydrophiles, les filtres organiques lipophiles et leurs mélanges.

14. Utilisation selon l'une quelconque des revendications précédentes, **caractérisée en ce que** le filtre UV organique est choisi parmi les dérivés de l'acide para-aminobenzoique ; les dérivés salicyliques ; les dérivés du dibenzoyiméthane ; les dérivés cinnamiques ; les dérivés de β,β-diphénylacrylate ; les dérivés de la benzophénone ; les dérivés du benzylidène camphre ; les dérivés du phenyl benzimidazole ; les dérivés de la triazine ; les dérivés du phenyl benzotriazole ; les dérivés anthraniliques ; les dérivés d'imidazolines ; les dérivés du benzalmalonate ; et leurs mélanges.

15. Utilisation selon l'une quelconque des revendications précédentes, **caractérisée en ce que** le filtre UV organique est choisi parmi le salicylate d'éthyl hexyle, le butyl méthoxydibenzoylmethane, l'éthylhexyl méthoxycinnamate, l'octocrylène, l'acide phenylbenzimidazole sulfonique, l'acide téréphthalylidène dicamphosulfonique, la benzophenone-3, la benzophenone-4, la benzophenone-5, le 4-méthylbenzylidène camphre, le benzimidazilate, l'anisotriazine, l'éthylhexyl triazone, le diéthylhexyl butamido triazone, le méthylène bis-benzotriazolyl tétraméthylbutylphenol, le Drometrizole trisiloxane, et leurs mélanges.

16. Utilisation selon l'une quelconque des revendications précédentes, **caractérisée en ce que** la quantité de filtre(s) organique(s) va de 0,1 à 25 % en poids, de préférence de 1 à 20 % en poids par rapport au poids total de la composition.

17. Utilisation selon l'une quelconque des revendications précédentes, **caractérisée en ce que** la composition comprend en outre un filtre physique choisi parmi les pigments et nano-pigments d'oxydes métalliques enrobés ou non enrobés.

18. Utilisation selon la revendication précédente, **caractérisée en ce que** lesdits pigments ou nanopigments sont choisis parmi les oxydes de titane, de zinc, de fer, de zirconium, de cérium et leurs mélanges, enrobés ou non.

19. Utilisation selon la revendication 17 ou 18, **caractérisée en ce que** la quantité de filtre(s) physique(s) va de 0,1 à 20 % en poids, de préférence de 0,5 à 12 % en poids par rapport au poids total de la composition.

20. Utilisation selon l'une quelconque des revendications précédentes, **caractérisée en ce que** la phase grasse de la composition comprend au moins une huile choisie parmi les huiles hydrocarbonées d'origine animale, les huiles hydrocarbonées d'origine végétale, les esters et les éthers de synthèse, les hydrocarbures linéaires ou ramifiés, les alcools gras ayant de 8 à 26 atomes de carbone, les alcools gras alcoxylés, les huiles fluorées partiellement hydrocarbonées et/ou siliconées, les huiles de silicone, et leurs mélanges.

21. Utilisation selon l'une quelconque des revendications précédentes, **caractérisée en ce que** la composition se présente sous forme d'une solution huileuse, d'une émulsion, d'un gel huileux, d'un produit anhydre liquide, pâteux ou solide, ou d'une dispersion d'une phase grasse dans une phase aqueuse à l'aide de sphérules.

22. Composition à application topique, **caractérisée en ce qu'**elle comprend, dans un milieu physiologiquement acceptable, (1) au moins une phase grasse liquide, (2) au moins un filtre UV organique et (3) au moins un polymère semi-cristallin solide à température ambiante et ayant une température de fusion inférieure à 70°C comportant a) un squelette polymérique et b) au moins une chaîne organique latérale cristallisable et/ou une séquence organique cristallisable faisant partie du squelette dudit polymère, ledit polymère ayant une masse moléculaire moyenne en nombre supérieure ou égale à 2 000, et ledit filtre UV n'étant pas un dérivé de dibenzoylméthane.

23. Composition selon la revendication précédente, **caractérisée en ce que** le polymère a une masse moléculaire moyenne en nombre allant de 3 000 à 500 000 et mieux de 4 000 à 99 000.

24. Composition selon l'une quelconque des revendications 22 ou 23, **caractérisée en ce que** le polymère est soluble dans la phase grasse à au moins 1 % en poids à une température supérieure à sa température de fusion.

25. Composition selon l'une quelconque des revendications 22 à 24, **caractérisée en ce que** le polymère a une température de fusion pF telle que 30°C ≤ pF < 50°C.

26. Composition selon l'une quelconque des revendications 22 à 25, **caractérisée en ce que** le polymère est choisi parmi :
- les copolymères séquencés de polyoléfines à cristallisation contrôlée,
- les polycondensats polyesters aliphatiques ou aromatiques et les copolyesters aliphatiques/aromatiques,
- les homo- ou co-polymères portant au moins une chaîne latérale cristallisable, et les homo- ou co-polymères portant dans le squelette au moins une séquence cristallisable,
- les homo- ou co-polymères portant au moins une chaîne latérale cristallisable à groupement(s) fluoré(s),
- et leurs mélanges.

27. Composition selon l'une quelconque des revendications 22 à 26, **caractérisée en ce que** le polymère est choisi parmi les homopolymères et copolymères comportant de 50 à 100% en poids de motifs résultant de la polymérisation d'un ou de plusieurs monomères porteurs de chaîne(s) latérale(s) hydrophobe(s) cristallisable(s).

28. Composition selon l'une quelconque des revendications 22 à 27, **caractérisée en ce que** le polymère est choisi parmi les homopolymères et copolymères résultant de la polymérisation d'au moins un monomère à chaîne(s) cristallisable(s) de formule X : 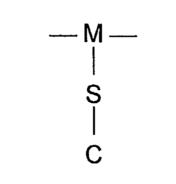 où M représente un atome du squelette polymérique, S représente un espaceur et C représente un groupe cristallisable.

29. Composition selon l'une quelconque des revendications 22 à 28, **caractérisée en ce que** le polymère est choisi parmi les homopolymères et copolymères résultant de la polymérisation d'au moins un monomère à chaîne cristallisable choisi parmi les (méth)acrylates d'alkyle saturés en C₁₄-C₂₄, les (méth)acrylates de perfluoroalkyle en C₁₁-C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, et leurs mélanges.

30. Composition selon l'une quelconque des revendications 22 à 29, **caractérisée en ce que** le polymère est choisi parmi les homopolymères d'alkyl(méth)acrylate ou d'alkyl(méth)acrylamide en C₁₄ à C₂₄ ; des copolymères de ces monomères avec un monomère hydrophile ; et leurs mélanges.

31. Composition selon l'une quelconque des revendications 22 à 30, **caractérisée en ce que** le polymère est choisi parmi les copolymères d'alkyl(méth)acrylate ou d'alkyl (méth)acrylamide avec un groupe alkyle en C₁₄ à C₂₄, avec la N-vinylpyrrolidone, l'hydroxyéthyl (méth)acrylate ; ou leurs mélanges.

32. Composition selon l'une quelconque des revendications 22 à 31, **caractérisée en ce que** le polymère représente de 0,1 à 50 % en poids de matière active, et de préférence de 0,5 à 20 % en poids de matière active par rapport au poids total de la composition.

33. Composition selon l'une quelconque des revendications 22 à 32, **caractérisée en ce que** le filtre UV organique est choisi parmi les filtres organiques hydrophiles, les filtres organiques lipophiles et leurs mélanges.

34. Composition selon l'une quelconque des revendications 22 à 33, **caractérisée en ce que** le filtre UV organique est choisi parmi les dérivés de l'acide para-aminobenzoique ; les dérivés salicyliques ; les dérivés cinnamiques ; les dérivés de β,β-diphénylacrylate; les dérivés de la benzophénone ; les dérivés du benzylidène camphre ; les dérivés du phenyl benzimidazole ; les dérivés de la triazine ; les dérivés du phenyl benzotriazole ; les dérivés anthraniliques ; les dérivés d'imidazolines ; les dérivés du benzalmalonate ; et leurs mélanges.

35. Composition selon l'une quelconque des revendications 22 à 34, **caractérisée en ce que** le filtre UV organique est choisi parmi le salicylate d'éthyl hexyle, l'éthylhexyl méthoxycinnamate, l'octocrylène, l'acide phenylbenzimidazole sulfonique, l'acide téréphthalylidène dicamphosulfonique, la benzophenone-3, la benzophenone-4, la benzophenone-5, le 4-méthylbenzylidène camphre, le benzimidazilate, l'anisotriazine, l'éthylhexyl triazone, le diéthylhexyl butamido triazone, le méthylène bis-benzotriazolyl tétraméthylbutylphenol, le Drometrizole trisiloxane, et leurs mélanges.

36. Composition selon l'une quelconque des revendications 22 à 35, **caractérisée en ce que** la quantité de filtre(s) organique(s) va de 0,1 à 25 % en poids, de préférence de 1 à 20 % en poids par rapport au poids total de la composition.

37. Composition selon l'une quelconque des revendications 22 à 36, **caractérisée en ce qu'**elle comprend en outre un filtre physique choisi parmi les pigments et nano-pigments d'oxydes métalliques enrobés ou non enrobés.

38. Composition selon la revendication précédente, **caractérisée en ce que** lesdits pigments ou nanopigments sont choisis parmi les oxydes de titane, de zinc, de fer, de zirconium, de cérium et leurs mélanges, enrobés ou non.

39. Composition selon la revendication 37 ou 38, **caractérisée en ce que** la quantité de filtre(s) physique(s) va de 0,1 à 20 % en poids, de préférence de 0,5 à 12 % en poids par rapport au poids total de la composition.

40. Composition selon l'une quelconque des revendications 22 à 39, **caractérisée en ce que** la phase grasse comprend au moins une huile choisie parmi les huiles hydrocarbonées d'origine animale, les huiles hydrocarbonées d'origine végétale, les esters et les éthers de synthèse, les hydrocarbures linéaires ou ramifiés, les alcools gras ayant de 8 à 26 atomes de carbone, les alcools gras alcoxylés, les huiles fluorées partiellement hydrocarbonées et/ou siliconées, les huiles de silicone, et leurs mélanges.

41. Composition selon l'une quelconque des revendications 22 à 40, **caractérisée en ce qu'**elle constitue une composition cosmétique.

42. Utilisation d'une composition telle que définie selon l'une quelconque des revendications 22 à 40, pour la fabrication d'une composition destinée à la protection de la peau et/ou des cheveux contre les effets néfastes pour la santé de la peau des rayonnements UV, en particulier le rayonnement solaire.
